# EUROPEAN PATENT APPLICATION

(11) **EP 4 299 571 A1**
(43) Date of publication of application: **03.01.2024**
(21) Application number: 22181685.3
(22) Date of filing: 28.06.2022
(51) Int. Cl.: C07D 339/08

(54) **A BICYCLOPENTYL THIANTHRENIUM COMPOUND, PROCESS FOR PREPARING THE SAME AND THE USE THEREOF**

(71) Applicant: Studiengesellschaft Kohle gGmbH, 45470 Mülheim (DE)
(72) Inventor: RITTER, Tobias, 45470 Muelheim (DE); BAI, Zibo, 45470 Mülheim an der Ruhr (DE); ALVAREZ PARI, Eva Maria Wara, 45470 Mülheim an der Ruhr (DE)

(57) **Abstract**

The present inventions discloses a novel bicyclopentyl thianthrenium compound referred to as TT-BCP ⁺ X ⁻ , a process for preparing the same and the use thereof for bicyclopentylating organic compounds.

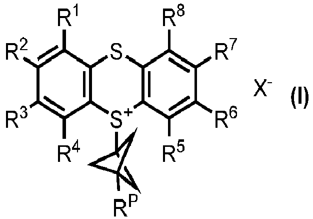

## Description

The present inventions refers a novel bicyclopentyl thianthrenium compound referred to as TT-BCP⁺X⁻, a process for preparing the same and the use thereof for bicyclopentylating organic compounds.

Bicyclopentanes (BCPs) are three dimensional isosteres of phenyl rings and, when 1,3-disubstituted, provide two exit vectors that are opposing each other, as in 1,4-disubstituted arenes. About 45% of marketed small molecule drugs contain phenyl substituents. Replacement of aryl substituents with 1,3-disubstituted bicyclopentanes can offer improvement of metabolic and pharmacokinetic properties of drug candidates; for example, the replacement of the fluorobenzene motif with BCP in the γ-secretase inhibitor BMS-708163 led to an increase of the aqueous solubility and metabolic stability over the parent compound as determined by in vivo mouse models. Currently, there are two main approaches to access 1,3-disubstituted BCPs within molecules of interest: Use of highly reactive [1.1.1]propellane as a starting material, which must be prepared before use because it has limited shelf stability even at -20 °C, and functionalized BCPs for alkylation. Several impressive examples that employ [1.1.1]propellane, for example for N-alkylation or even difunctionalization, have been advanced over the recent past. Despite the large synthetic utility of the products, all the synthetic routes have in common that [1.1.1]propellane is not suitable for central production and distribution and therefore of attenuated utility for practitioners. A more practical reagent of similar or greater utility could increase the occurrences of BCP substituent incorporation to benefit from their desirable properties but such a reagent class has not yet been reported. Several useful BCP-based reagents such as Grignard reagents, iodides, boronates, and redox-active esters, have been reported in the prior art. Most such reagents can successfully engage in C-C bond formations, yet, none has reached the generality in reactivity of [1.1.1]propellane, and they often lack stability for storage or require multiple steps for preparation. To date, no BCP-based reagents nor [1.1.1]propellane-based reactivity are available for aryl BCP ether synthesis.

In nature, sulfonium salts can act as efficient alkylation reagents. Similarly, chemists have made use of alkylation reactions based on sulfonium salts but the transfer of tertiary alkyl groups, such as bicyclopentyl, remains unknown. The inventors have previously reported new reactivity of arylthianthrenium (TT) salts that can expand the chemical space in comparison to other (pseudo)halides and be rationalized by the unusual properties of the thianthrene scaffold. Based on the single electron reactivity, the high reduction potential, and the ability to function as good leaving group and readily engage in radical chemistry, the inventors devised a synthesis of BCP-thianthrenium salts to function as readily available, stable, and versatile alkylating reagents. Thianthrenium-substituted BCPs can engage in radical chemistry, distinct from that of conventional alkyl sulfonium salts, that productively combines photoredox catalysis with transition-metal-mediated bond formation. While copper catalysis has been productive for thianthrene- and BCP-based chemistry, the inventors also introduce here previously unreported photoredox mediated nickel catalyzed cross-coupling with thianthrenium salts.

Because of its simple preparation, handling, high reactivity, and broad tolerance of functional groups present in complex molecules, as well as its divergent reactivity, the inventors expect that TT-BCP⁺X⁻, in particular TT-BCP⁺ BF₄⁻ will find widespread utility in future reaction chemistry development. The main difference of the inventive TT-BCP⁺X⁻when compared to most other sulfonium-based reagents is its simple one-step synthesis protocol; in contrast, the practical synthesis for the classical *Umemoto's* reagent requires nine steps. The fundamental difference to the *Togni* reagents is the higher reduction potential of the inventive TT-CF₃⁺X⁻, a consequence of the positive charge that can result in complementary reactivity in single electron transfer reactions when compared to the λ³-iodane compounds.

Thus, the present invention is directed to an optionally substituted bicyclopentyl thianthrenium derivative TT-BCP⁺X⁻. In more detail, the present invention is thus directed, in a first aspect, to a thianthrene derivative of the Formula (I): 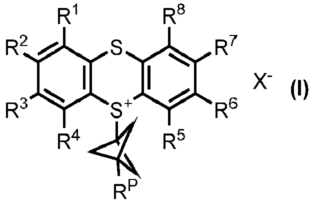 wherein R¹ to R⁸ may be the same or different and are each selected from hydrogen, halogen, a C₁ to C₆ alkyl group, which is optionally substituted by at least one halogen, or a -O-C₁ to C₆ alkyl group, wherein R^{P} represents CF₃ or CN, and wherein X⁻ is an anion, selected from F-, Cl⁻, triflate-, BF₄⁻, SbF₆⁻, PF₆⁻, ClO₄⁻, 0.5 SO₄²⁻ or NO₃.

In an embodiment of the thianthrene derivative of the Formula (I), R¹ to R⁸ may be the same or different and are each selected from hydrogen, Cl or F, R^{p} is as defined in claim 1 and X⁻is an anion as defined before, preferably triflate, or BF₄.

In another embodiment of the thianthrene derivative of the Formula (I), R², R³, R⁶ and R⁷ are selected from -OCH₃, F or CF₃ and the others of R¹ to R⁸ are hydrogen, RP is as defined in claim 1 and X⁻ is an anion as defined before, preferably a triflate or BF₄ anion.

In yet another embodiment of the thianthrene derivative of the Formula (I), R¹ to R⁸ are hydrogen, RP is as defined in claim 1 and X⁻ is an anion as defined before, preferably a triflate or BF₄ anion

In the above formulae, X⁻ represents an anion selected from F⁻, Cl⁻, triflate- , BF₄⁻, SbF₆⁻, PF₆⁻, ClO₄⁻, 0.5 SO₄²⁻, or NO₃⁻, and similar anions which result in a stable ion pair with the bicyclopentyl thianthrenium cation.

The inventive TT-BCP⁺X⁻ is useful for bicyclopentylating organic compounds selected from aryl halogenides, phenols, and nucleophilic compounds including N-nucleophiles. Thus, the present invention is furthermore directed, in a second aspect, to the use of a bicyclopentyl thianthrenium compound of the Formula (I) as a transfer agent for transferring a bicyclopentyl group to an organic compound selected from aryl halogenides, phenols, and nucleophilic compounds including N-nucleophiles, which is optionally substituted by at least group selected from hydroxyl, aldehyde, carboxylic acid ester, olefin, amino, amido, sulfonamido, halogen such as bromo, fluoro, chloro,

Thus, the inventive bicyclopentyl thianthrenium compound of the Formula (I) can be used as a transfer agent for transferring a bicyclopentyl group under irradiation in the presence of a photocatalyst in a transition-metal-mediated bond formation to an organic compound selected from aryl halogenides, phenols, and nucleophilic compounds including N-nucleophiles which reaction includes alkylation of phenols, N-heterocycles, amines, amides, sulfonamides, anilines, arenes, heteroarenes and haloarenes.

In the inventive process for preparing the inventive TT-BCP⁺X⁻ or the use thereof for bicyclopentylating, the choice of the organic solvent is not critical as long as it is an aprotic organic solvent selected from acetonitrile, other nitriles, chlorinated hydrocarbons, or other aprotic solvents, or mixtures thereof. The reaction conditions are also not critical and the reaction is usually carried out at a temperature between -78°C and 50°C, preferably 0°C to 30°C, under ambient pressure and optionally under an inert gas atmosphere.

In the inventive process for transferring the bicyclopentyl group, the organic compound may be a monocyclic or polycyclic, aromatic or heteroaromatic hydrocarbon ring structure having 5 to 22 carbon atoms, which may be unsubstituted or substituted by one of more substituents selected from saturated or unsaturated, straight chain or branched aliphatic hydrocarbons having 1 to 20 carbon atoms, aromatic or heteroaromatic hydrocarbons having 5 to 22 carbon atoms, heterosubstituents, or which may be part of a cyclic hydrocarbon ring system (carbocyclic) with 5 to 30 carbon atoms and/or heteroatoms. The definition for said aliphatic hydrocarbons may include one or more heteroatoms such O, N, S in the hydrocarbon chain.

In the context of the aspects of the present invention, the following definitions are more general terms which are used throughout the present application.

When a range of values is listed, it is intended to encompass each value and sub-range within the range. For example "C₁₋₆" is intended to encompass, C₁, C₂, C₃, C₄, C₅, C₆, C₁₋₆, C₁₋₅, C₁₋₄, C₁₋₃, C₁₋₂, C₂₋₆, C₂₋₅, C₂₋₄, C₂₋₃, C₃₋₆, C₃₋₅, C₃₋₄, C₄₋₆, C₄₋₅, and C₅₋₆.

The term "aliphatic" includes both saturated and unsaturated, straight chain (*i.e.,* unbranched), branched, acyclic, cyclic, or polycyclic aliphatic hydrocarbons, which are optionally substituted with one or more functional groups. As will be appreciated by one of ordinary skill in the art, "aliphatic" is intended herein to include, but is not limited to, alkyl, alkenyl, alkynyl, cycloalkyl, cycloalkenyl, and cycloalkynyl moieties. Thus, the term "alkyl" includes straight, branched and cyclic alkyl groups. An analogous convention applies to other generic terms such as "alkenyl", "alkynyl", and the like. Furthermore, the terms "alkyl", "alkenyl", "alkynyl", and the like encompass both substituted and unsubstituted groups. In certain embodiments, "lower alkyl" is used to indicate those alkyl groups (cyclic, substituted, unsubstituted, branched or unbranched) having 1-6 carbon atoms.

As used herein, "alkyl" refers to a radical of a straight-chain, branched or cyclic saturated hydrocarbon group having from 1 to 20 carbon atoms ("C₁₋₂₀ alkyl"). In some embodiments, an alkyl group has 1 to 10 carbon atoms ("C₁₋₁₀ alkyl"). In some embodiments, an alkyl group has 1 to 9 carbon atoms ("C₁₋₉ alkyl"). In some embodiments, an alkyl group has 1 to 8 carbon atoms ("C₁₋₈ alkyl"). In some embodiments, an alkyl group has 1 to 7 carbon atoms ("C₁₋₇ alkyl"). In some embodiments, an alkyl group has 1 to 6 carbon atoms ("C₁₋₆ alkyl"). In some embodiments, an alkyl group has 1 to 5 carbon atoms ("C₁₋₅ alkyl"). In some embodiments, an alkyl group has 1 to 4 carbon atoms ("C₁₋₄ alkyl"). In some embodiments, an alkyl group has 1 to 3 carbon atoms ("C₁₋₃ alkyl"). In some embodiments, an alkyl group has 1 to 2 carbon atoms ("C₁₋₂ alkyl"). In some embodiments, an alkyl group has 1 carbon atom ("C₁ alkyl"). In some embodiments, an alkyl group has 2 to 6 carbon atoms ("C₂₋₆ alkyl"). Examples of C₁₋₆ alkyl groups include methyl (C₁), ethyl (C₂), n-propyl (C₃), isopropyl (C3), n-butyl (C₄), tert-butyl (C₄), sec-butyl (C₄), iso-butyl (C₄), n-pentyl (C₅), 3-pentanyl (C₅), amyl (C₅), neopentyl (C₅), 3-methyl-2-butanyl (C₅), tertiary amyl (C₅), and n-hexyl (C₆). Additional examples of alkyl groups include n-heptyl (C₇), n-octyl (C₈) and the like. Unless otherwise specified, each instance of an alkyl group is independently unsubstituted (an "unsubstituted alkyl") or substituted (a "substituted alkyl") with one or more substituents. In certain embodiments, the alkyl group is an unsubstituted C₁₋₁₀ alkyl (e.g., -CH₃). In certain embodiments, the alkyl group is a substituted C₁₋₁₀ alkyl.

"Aryl" or aromatic hydrocarbon refers to a radical of a monocyclic or polycyclic (e.g., bicyclic or tricyclic) 4n+2 aromatic ring system (e.g., having 6, 10, or 14 pi electrons shared in a cyclic array) having 6-14 ring carbon atoms and zero heteroatoms provided in the aromatic ring system ("C₆₋₁₄ aryl"). In some embodiments, an aryl group has six ring carbon atoms ("C₆ aryl"; e.g., phenyl). In some embodiments, an aryl group has ten ring carbon atoms ("C₁₀ aryl"; e.g., naphthyl such as 1-naphthyl and 2-naphthyl). In some embodiments, an aryl group has fourteen ring carbon atoms ("C₁₄ aryl"; e.g., anthracyl). "Aryl" also includes ring systems wherein the aryl ring, as defined above, is fused with one or more carbocyclyl or heterocyclyl groups wherein the radical or point of attachment is on the aryl ring, and in such instances, the number of carbon atoms continue to designate the number of carbon atoms in the aryl ring system. Unless otherwise specified, each instance of an aryl group is independently optionally substituted, *i.e.,* unsubstituted (an "unsubstituted aryl") or substituted (a "substituted aryl") with one or more substituents. In certain embodiments, the aryl group is unsubstituted C₆₋₁₄ aryl. In certain embodiments, the aryl group is substituted C₆₋₁₄ aryl.

"Aralkyl" is a subset of alkyl and aryl and refers to an optionally substituted alkyl group substituted by an optionally substituted aryl group. In certain embodiments, the aralkyl is optionally substituted benzyl. In certain embodiments, the aralkyl is benzyl. In certain embodiments, the aralkyl is optionally substituted phenethyl. In certain embodiments, the aralkyl is phenethyl.

"Heteroaryl" or heteroaromatic hydrocarbon refers to a radical of a 5-14 membered monocyclic or bicyclic 4n+2 aromatic ring system (e.g., having 6 or 10 pi electrons shared in a cyclic array) having ring carbon atoms and 1-4 ring heteroatoms provided in the aromatic ring system, wherein each heteroatom is independently selected from nitrogen, oxygen, and sulfur ("5-14 membered heteroaryl"). In heteroaryl groups that contain one or more nitrogen atoms, the point of attachment can be a carbon or nitrogen atom, as valency permits. Heteroaryl bicyclic ring systems can include one or more heteroatoms in one or both rings. "Heteroaryl" includes ring systems wherein the heteroaryl ring, as defined above, is fused with one or more carbocyclyl or heterocyclyl groups wherein the point of attachment is on the heteroaryl ring, and in such instances, the number of ring members continue to designate the number of ring members in the heteroaryl ring system. "Heteroaryl" also includes ring systems wherein the heteroaryl ring, as defined above, is fused with one or more aryl groups wherein the point of attachment is either on the aryl or heteroaryl ring, and in such instances, the number of ring members designates the number of ring members in the fused (aryl/heteroaryl) ring system. Bicyclic heteroaryl groups wherein one ring does not contain a heteroatom (e.g., indolyl, quinolinyl, carbazolyl, and the like) the point of attachment can be on either ring, *i.e.,* either the ring bearing a heteroatom *(e.g.,* 2-indolyl) or the ring that does not contain a heteroatom (e.g., 5-indolyl).

In some embodiments, a heteroaryl group is a 5-10 membered aromatic ring system having ring carbon atoms and 1-4 ring heteroatoms provided in the aromatic ring system, wherein each heteroatom is independently selected from nitrogen, oxygen, and sulfur ("5-10 membered heteroaryl"). In some embodiments, a heteroaryl group is a 5-8 membered aromatic ring system having ring carbon atoms and 1-4 ring heteroatoms provided in the aromatic ring system, wherein each heteroatom is independently selected from nitrogen, oxygen, and sulfur ("5-8 membered heteroaryl"). In some embodiments, a heteroaryl group is a 5-6 membered aromatic ring system having ring carbon atoms and 1-4 ring heteroatoms provided in the aromatic ring system, wherein each heteroatom is independently selected from nitrogen, oxygen, and sulfur ("5-6 membered heteroaryl"). In some embodiments, the 5-6 membered heteroaryl has 1-3 ring heteroatoms selected from nitrogen, oxygen, and sulfur. In some embodiments, the 5-6 membered heteroaryl has 1-2 ring heteroatoms selected from nitrogen, oxygen, and sulfur. In some embodiments, the 5-6 membered heteroaryl has 1 ring heteroatom selected from nitrogen, oxygen, and sulfur.

Unless otherwise specified, each instance of a heteroaryl group is independently optionally substituted, *i.e.,* unsubstituted (an "unsubstituted heteroaryl") or substituted (a "substituted heteroaryl") with one or more substituents. In certain embodiments, the heteroaryl group is unsubstituted 5-14 membered heteroaryl. In certain embodiments, the heteroaryl group is substituted 5-14 membered heteroaryl.

Exemplary 5-membered heteroaryl groups containing one heteroatom include, without limitation, pyrrolyl, furanyl and thiophenyl. Exemplary 5-membered heteroaryl groups containing two heteroatoms include, without limitation, imidazolyl, pyrazolyl, oxazolyl, isoxazolyl, thiazolyl, and isothiazolyl. Exemplary 5-membered heteroaryl groups containing three heteroatoms include, without limitation, triazolyl, oxadiazolyl, and thiadiazolyl. Exemplary 5-membered heteroaryl groups containing four heteroatoms include, without limitation, tetrazolyl. Exemplary 6-membered heteroaryl groups containing one heteroatom include, without limitation, pyridinyl. Exemplary 6-membered heteroaryl groups containing two heteroatoms include, without limitation, pyridazinyl, pyrimidinyl, and pyrazinyl. Exemplary 6-membered heteroaryl groups containing three or four heteroatoms include, without limitation, triazinyl and tetrazinyl, respectively. Exemplary 7-membered heteroaryl groups containing one heteroatom include, without limitation, azepinyl, oxepinyl, and thiepinyl. Exemplary 5,6-bicyclic heteroaryl groups include, without limitation, indolyl, isoindolyl, indazolyl, benzotriazolyl, benzothiophenyl, isobenzothiophenyl, benzofuranyl, benzoisofuranyl, benzimidazolyl, benzoxazolyl, benzisoxazolyl, benzoxadiazolyl, benzthiazolyl, benzisothiazolyl, benzthiadiazolyl, indolizinyl, and purinyl. Exemplary 6,6-bicyclic heteroaryl groups include, without limitation, naphthyridinyl, pteridinyl, quinolinyl, isoquinolinyl, cinnolinyl, quinoxalinyl, phthalazinyl, and quinazolinyl.

"Heteroaralkyl" is a subset of alkyl and heteroaryl and refers to an optionally substituted alkyl group substituted by an optionally substituted heteroaryl group.

"Unsaturated" or "partially unsaturated" refers to a group that includes at least one double or triple bond. A "partially unsaturated" ring system is further intended to encompass rings having multiple sites of unsaturation, but is not intended to include aromatic groups (e.g., aryl or heteroaryl groups) as herein defined. Likewise, "saturated" refers to a group that does not contain a double or triple bond, *i.e.,* contains all single bonds.

Alkyl, alkenyl, alkynyl, carbocyclyl, heterocyclyl, aryl, and heteroaryl groups, which are divalent bridging groups, are further referred to using the suffix -ene, e.g., alkylene, alkenylene, alkynylene, carbocyclylene, heterocyclylene, arylene, and heteroarylene.

An atom, moiety, or group described herein may be unsubstituted or substituted, as valency permits, unless otherwise provided expressly. The term "optionally substituted" refers to substituted or unsubstituted.

Alkyl, alkenyl, alkynyl, carbocyclyl, heterocyclyl, aryl, and heteroaryl groups are optionally substituted (e.g., "substituted" or "unsubstituted" alkyl, "substituted" or "unsubstituted" alkenyl, "substituted" or "unsubstituted" alkynyl, "substituted" or "unsubstituted" carbocyclyl, "substituted" or "unsubstituted" heterocyclyl, "substituted" or "unsubstituted" aryl or "substituted" or "unsubstituted" heteroaryl group). In general, the term "substituted", whether preceded by the term "optionally" or not, means that at least one hydrogen present on a group *(e.g.,* a carbon or nitrogen atom) is replaced with a permissible substituent, *e.g.,* a substituent which upon substitution results in a stable compound, e.g., a compound which does not spontaneously undergo transformation such as by rearrangement, cyclization, elimination, or other reaction. Unless otherwise indicated, a "substituted" group has a substituent at one or more substitutable positions of the group, and when more than one position in any given structure is substituted, the substituent is either the same or different at each position. For purposes of this invention, heteroatoms such as nitrogen may have hydrogen substituents and/or any suitable substituent as described herein which satisfy the valencies of the heteroatoms and results in the formation of a stable moiety. In certain embodiments, the substituent is a carbon atom substituent. In certain embodiments, the substituent is a nitrogen atom substituent. In certain embodiments, the substituent is an oxygen atom substituent. In certain embodiments, the substituent is a sulfur atom substituent.

Exemplary substituents include, but are not limited to, halogen, -CN, -NO₂, -N₃, -SO₂H, - SO₃H, -OH, -O-alkyl, -N-dialkyl, -SH, -S.alkyl, -C(=O)alkyl,-CO₂H, -CHO.

"Halo" or "halogen" refers to fluorine (fluoro, -F), chlorine (chloro, -CI), bromine (bromo, - Br), or iodine (iodo, -I).

"Acyl" refers to a moiety selected from the group consisting of-C(=O)R^{aa},-CHO, -CO₂R^{aa}, -C(=O)N(R^{bb})₂, -C(=NR^{bb})R^{aa}, -C(=NR^{bb})OR^{aa}, -C(=NR^{bb})N(R^{bb})₂, -C(=O)NR^{bb}SO₂R^{aa}, - C(=S)N(R^{bb})₂, -C(=O)SR^{aa}, or-C(=S)SR^{aa}, wherein R^{aa} and R^{bb} are as defined herein.

The term "catalysis," "catalyze," or "catalytic" refers to the increase in rate of a reaction due to the participation of a substance called a "catalyst." In certain embodiments, the amount and nature of a catalyst remains essentially unchanged during a reaction. In certain embodiments, a catalyst is regenerated, or the nature of a catalyst is essentially restored after a reaction. A catalyst may participate in multiple chemical transformations. The effect of a catalyst may vary due to the presence of other substances known as inhibitors or poisons (which reduce the catalytic activity) or promoters (which increase the activity). Catalyzed reactions have a lower activation energy (rate-limiting free energy of activation) than the corresponding uncatalyzed reaction, resulting in a higher reaction rate at the same temperature. Catalysts may affect the reaction environment favorably, or bind to the reagents to polarize bonds, or form specific intermediates that are not typically produced by a uncatalyzed reaction, or cause dissociation of reagents to reactive forms.

The invention is not intended to be limited in any manner by the above exemplary listing of substituents.

In conclusion, the inventors have developed a new bicyclopentylating reagent, bicyclopentyl thianthrenium triflate (1, TT-BCP⁺OTf⁻), which is easily accessible from readily available starting materials in a single step. The new reagent can engage in various reactions and promises to be of synthetic utility.

The invention is further illustrated by the attached Figures and Examples. In the Figures, the respective Figure shows:
- Figures 1A and 1B:: Bicyclo [1.1.1]pentane thianthrenium salts;
- Figures 2A and 2B:: Substrate scope for Cu-catalyzed C-O cross coupling of 1 and 2 with phenols;
- Figures 3A and 3B:: Substrate scope for C-N cross coupling of 1 with N-nucleophiles; and
- Figures 4A and 4B:: Substrate scope for Ni-catalyzed reductive C-C cross coupling of 1 with (het)aryl.

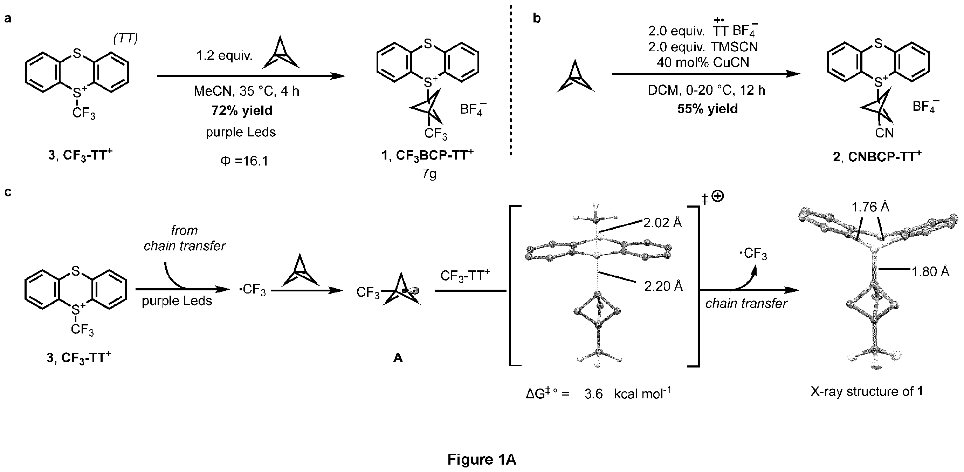

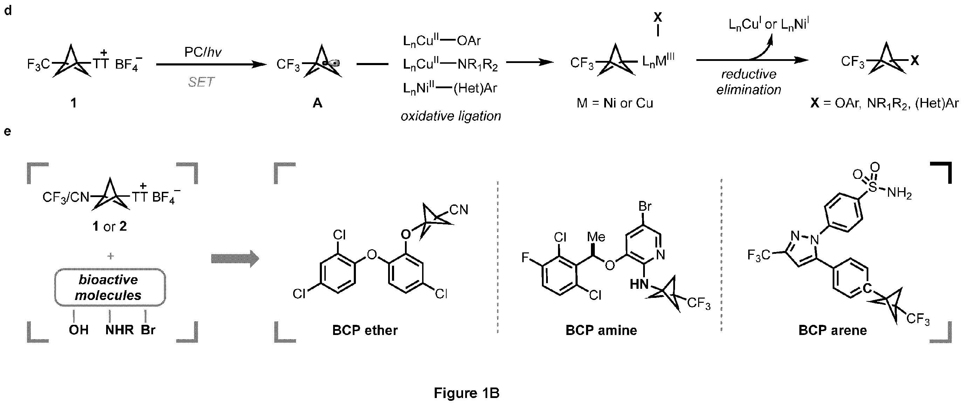

In more detail, Figure 1 illustrates:
a: Synthesis of CF₃BCP-TT⁺ BF₄⁻(1) salt and quantum yield. The general reaction conditions are as follows: CF₃-TT⁺ OTf⁻ (21 mmol, 1.0 equiv), [1.1.1]propellane solution in Et₂O (c = 0.85 M) (1.2 equiv.), MeCN (0.14 M), purple LED (40 W), 30 °C, 4 h, followed by aqueous workup with NaBF₄ (10% w/w).
b: Synthesis of CNBCP-TT⁺ BF₄⁻ (2) salt. Conditions: [1.1.1]propellane (1.0 equiv.), thianthrenium tetrafluoroborate (TT^{•+} BF₄⁻) (2.0 equiv.), TMSCN (2.0 equiv.), CuCN (40 mol%), DCM (0.20 M), 0-20 °C, 12 h.
c: Proposed mechanism of 1 formation proceeding through a radical chain propagation and crystal structure of 1. Thermal ellipsoids are drawn at the 50% probability level. Hydrogen atoms and counterion have been omitted for clarity.
d: Proposed mechanism for the cross-coupling of BCP-TT⁺ salts enabled by the photoredox catalysis and transition metal catalysis.
e: Synthetic utility of BCP-TT⁺ salts for C-O, C-N and C-C bond formation. TT, thianthrene; CF₃, trifluoromethyl; TMS, trimethylsilyl; PC, photoredox catalyst; Ar, aryl; Het, hetero; BCP, bicyclo[1.1.1]pent-1-yl.

As shown in the Figures, a practical synthesis of the CF₃BCP-TT⁺ salt 1 was accomplished by a simple addition reaction between the trifluoromethylthianthrenium reagent 3 and [1.1.1]propellane (Fig. 1a). All experimental observations (Supplementary Figs. S10-S11) and DFT calculations (Supplementary Tables. S15-S18) are consistent with a radical chain transfer mechanism that includes irradiation of 3 with blue LEDs at a wavelength transparent to 1 to induce homolytic S-CF₃ cleavage, followed by radical addition of CF₃ radical to [1.1.1]propellane to form putative BCP radical A. Subsequent chain propagation by TT radical cation abstraction from 3 by A through a low lying transition state TS is supported by DFT and consistent with a measured quantum yield of Φ = 16 and the stability of the TT radical cation, which sets thianthrene apart from conventional sulfides (Fig 1c). In contrast to volatile and thermally unstable propellane, compound 1 is a non-hygroscopic, free-flowing powder that can be stored under ambient conditions without observable decomposition for at least 5 months, and a melting point of 150 °C. A differential scanning calorimetry (DSC) analysis confirmed that heating of 1 up to 170 °C is not accompanied by any exothermic decomposition process, which attests to its favorable safety properties. Compound 1 is made from [1.1.1]propellane, yet, the practitioner interested in BCP substitution would not be required to handle the unstable reagent if 1 were produced centrally and distributed. The inventors also established that the class of BCP-TT⁺ compounds is not limited to 1, and a synthesis other than radical chain transfer from S-substituted thianthrene-based reagents can access the novel compound class, as shown by a synthesis from the persistent thianthrenium radical cation to afford the cyano-substituted BCP reagent 2 (Fig. 1b).

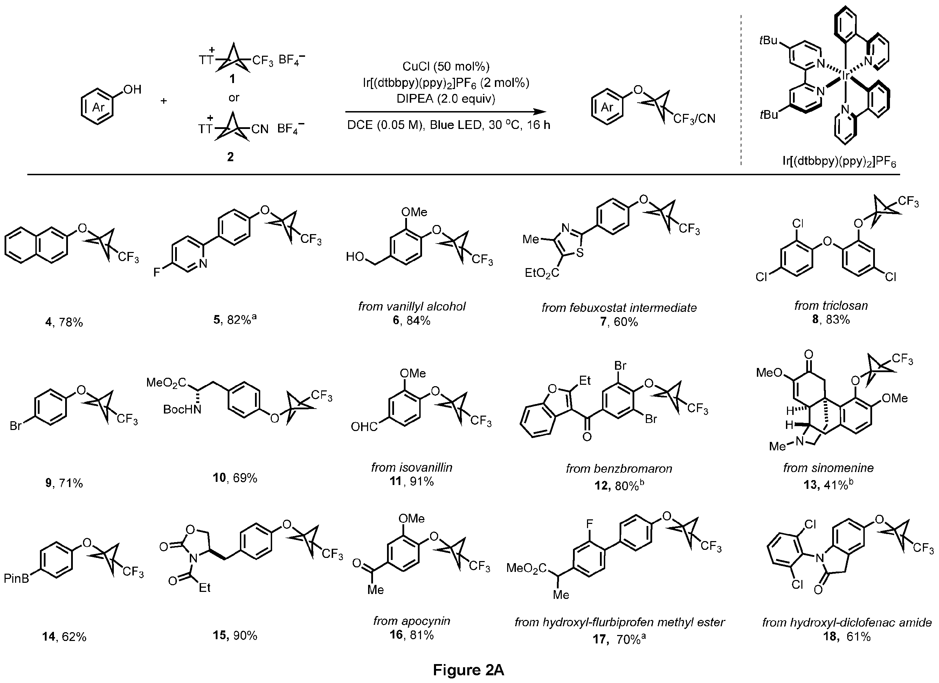

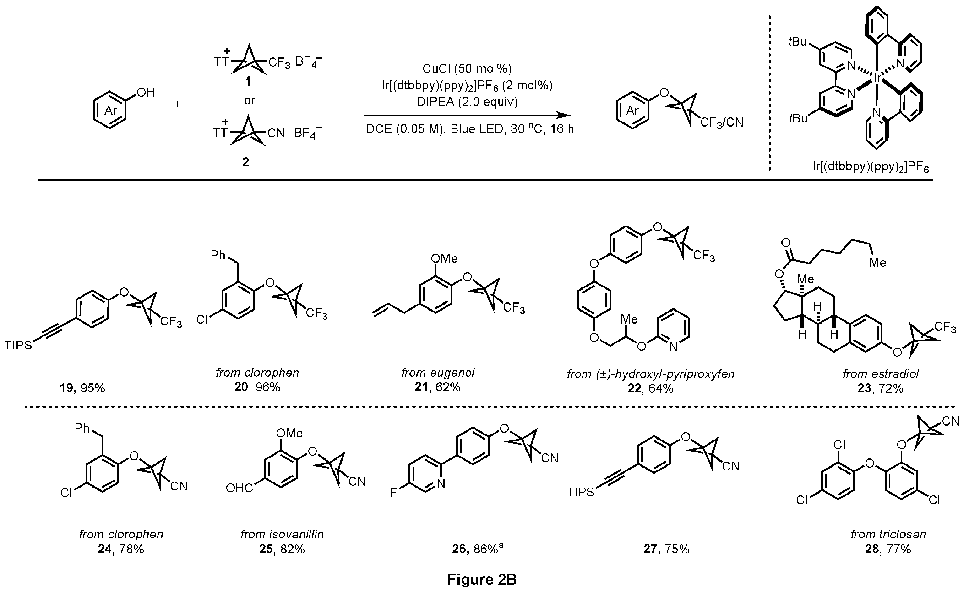

As shown in Figure 2, the general reaction conditions for Cu-catalyzed C-O cross coupling of 1 and 2 with phenols are as follows: phenol (0.15 mmol, 1.0 equiv), 1 or 2 (2.0 equiv.), Ir[(dtbbpy)(ppy)₂]PF₆ (2 mol%), CuCI (50 mol%), DIPEA (2.0 equiv.), DCE (0.05 M), blue LED (40 W), 30 °C, 16 h. ^{a}DCM (0.05 M) was used instead. ^{b}1 (3.0 equiv.) and CuCI (100 mol%) were used.

Aryl bicyclo[1.1.1]pentyl ethers have potential as bioisosteres for diaryl ether derivatives that are common structural motifs in natural and synthetic pharmaceutically important compounds. However, no synthesis to construct aryl BCP ethers is currently documented. Reagents **1** and **2** can successfully be employed in the metallaphotoredox-catalyzed alkylation of phenols with sub-stoichiometric amounts of copper salts to access the previously unknown class of aryl BCP ethers (Fig. 2). The reactions exhibits broad scope, and proceeds efficiently with phenols bearing electron-rich, -neutral, and -poor substituents (e.g. **6, 4,** and **12,** respectively), as well as ortho-substituted phenols (e.g. **6, 11, 13).** Synthetically useful functional groups such as hydroxyl, chloro, bromo, ester, amide, aldehyde, boronate, silyl, ketone, alkynyl, alkenyl, and even tertiary amines are tolerated. In addition, Lewis basic heterocycles, including pyridine and thiazole, that can be a liability in transition metal catalyzed coupling reactions, do not inhibit the desired cross coupling reactivity. The reaction is chemoselective with respect to N-nucleophiles (e.g. **10,** vide infra). Due to the large functional-group compatibility, late-stage functionalization of drug molecules, such as triclosan **(8),** benzbromaron **(12),** sinomenine **(13),** and clorophen **(20)** is accessible. Combined with thianthrene-mediated late-stage aromatic C-H hydroxylation, we have realized the formal site-selective C-H bicyclopentyloxylation of small-molecule pharmaceuticals, such as flurbiprofen methyl ester **(17),** diclofenac amide **(18),** and pyriproxyfen **(22).**

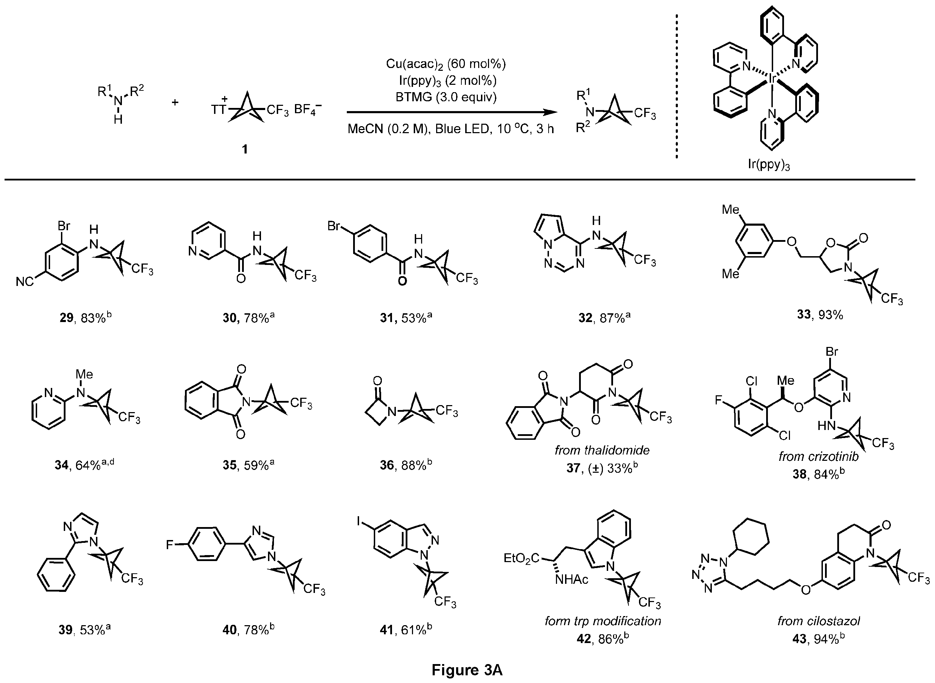

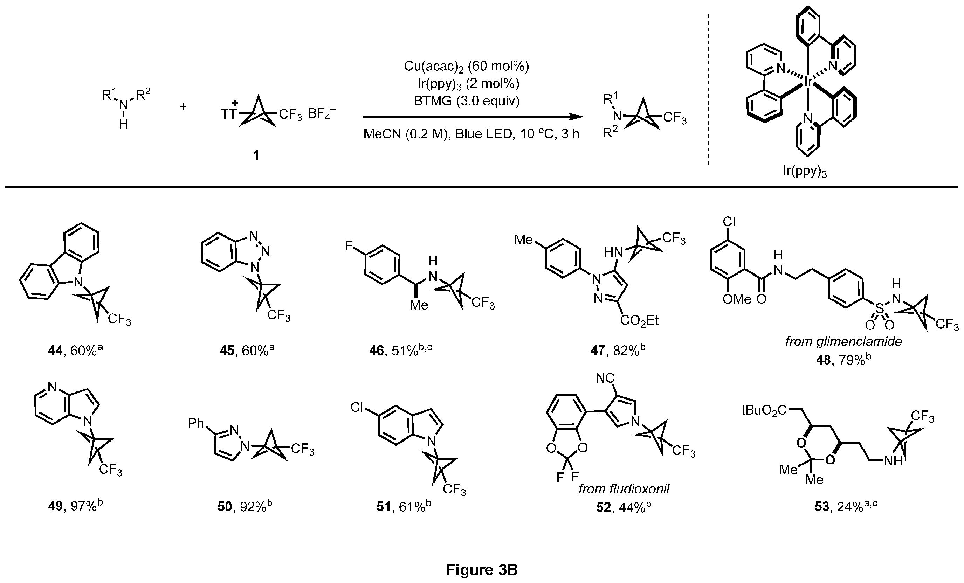

As illustrated in Figure 3, an analogous strategy is successful for bicyclopentylation of N-nucleophiles. Distinct from published procedures for N-alkylation reactions with propellane, bicyclopentylation with 1 proceeds with a larger scope with respect to the nitrogen nucleophile, including amines, anilines, amides, sulfonamides, 2-aminopyridines, imidazoles, triazines, 5-aminopyrazole, benzotriazoles (45), pyrroles (52), indoles (42, 51), azaindoles (49), carbazoles (44), indazoles (41), pyrazoles (50), and β-lactams (36). As in the corresponding ether bond formation, large functional group tolerance, even for redox-active aryl iodides (41), enables late-stage modification of various pharmaceuticals as shown in Figure 3. Basic, electron-rich tertiary amines are not tolerated, potentially a consequence of their single electron oxidation by excited photoredox catalysts. When more than one nitrogen nucleophile is present, functionalization of the more acidic position proceeds chemoselectively (e.g. 48). Both C-O and C-N bond forming reactions are, in principle, catalytic in transition metal, yet, use of about half an equivalent of copper afforded the highest yields.

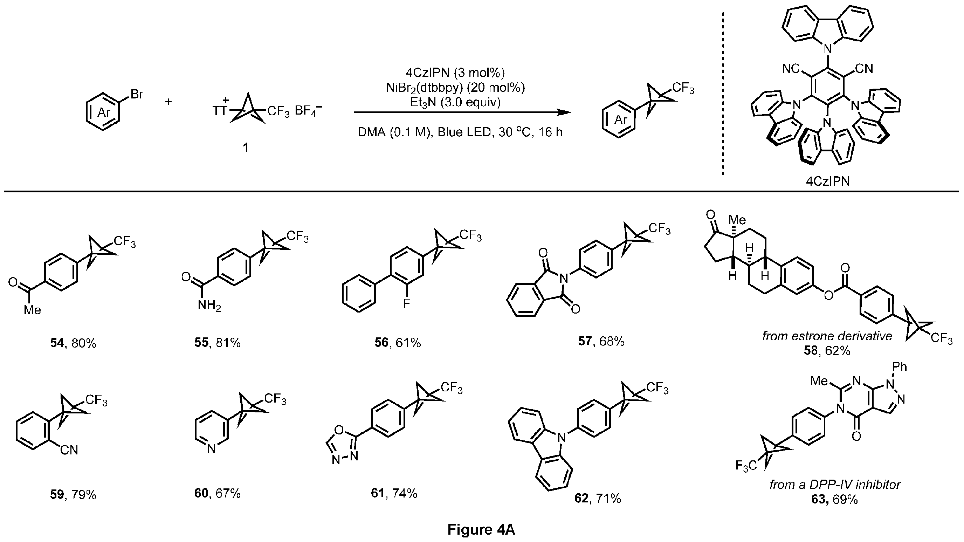

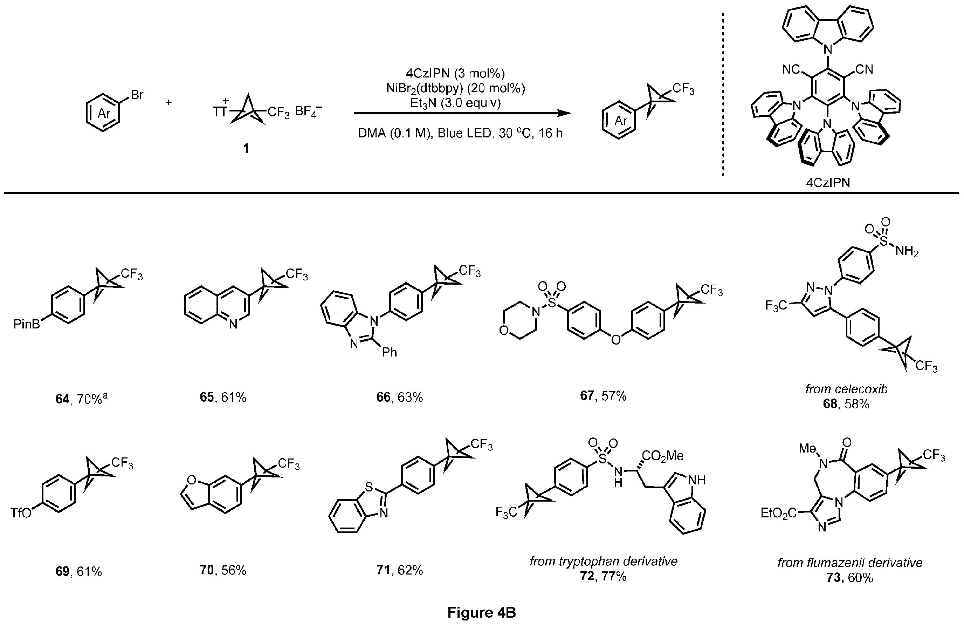

In addition to its simple synthesis and stability, reagent 1 can, beyond C-heteroatom cross coupling with copper, also participate in metallophotoredox catalysis with nickel catalysts for reductive C-C cross coupling reactions with (het)aryl bromides (Fig. 4). Synergistic cooperation of nickel catalysis and photoredox catalysis with thianthrenium salts has not been reported before. Carbon-carbon cross coupling reactions of iodo-BCPs, BCP Grignard reagents, BCP-boronates, and BCP redox active esters have been developed previously but not with a reagent as synthetically convenient as 1. A mechanism from 1 could proceed through a Ni(0-II-III-I) cycle with oxidative ligation of the BCP radical to a Ni(II) aryl complex obtained by oxidative addition of Ni(0) to an aryl bromide, with ensuing reductive C-C elimination form a putative high-valent Ni(III) complex. Aryl as well as heteroaryl bromides participate in the reaction, and the most prominent side reaction for electron-rich aryl bromides is proto-debromination. Functional group tolerance is high, including for example boronate (64) and triflate (69) substituents, for subsequent diversification, and the reaction can be used for late-stage functionalizations (e.g. 68).

As shown in Figure 4, the general reaction conditions are as follows: Aryl halide (0.20 mmol, 1.0 equiv), 1 (1.5 equiv.), 4CzIPN (3 mol%), Ni(dtbbpy)Br₂ (20 mol%), Et₃N (3.0 equiv.), DMA (0.1 M), blue LED (40 W), 30 °C, 16 h. ^{a} Ni(dtbbpy)Cl₂ (20 mol%) was used as catalyst. The inventors reported a storable, thianthrenium-based class of BCP-transfer reagents that can afford potentially valuable small molecules that are in part currently inaccessible by other methods. The inventors anticipate that commercial availability of a stable and readily employed reagent would enable practitioners, for example in the pharmaceutical industry, to introduce the promising BCP substituent substantially more straightforwardly into small molecules of interest than is possible today.

### Materials and Methods

All reactions were carried out under an ambient atmosphere unless otherwise stated and monitored by thin-layer chromatography (TLC). Air- and moisture-sensitive manipulations were performed using standard Schlenk- and glove-box techniques under an atmosphere of argon or dinitrogen. High-resolution mass spectra were obtained using Q Exactive Plus from Thermo. Concentration under reduced pressure was performed by rotary evaporation at 25-40 °C at an appropriate pressure. Purified compounds were further dried under high vacuum (0.010-0.005 mBar). Yields refer to purified and spectroscopically pure compounds, unless otherwise stated.

### Solvents

Anhydrous DCE and DMA were purchased from Acros Organics and Sigma Aldrich. Other anhydrous solvents were obtained from Phoenix Solvent Drying Systems. All deuterated solvents were purchased from Euriso-Top.

### Chromatography

Thin layer chromatography (TLC) was performed using EMD TLC plates pre-coated with 250 µm thickness silica gel 60 F₂₅₄ plates and visualized by fluorescence quenching under UV light and KMnO₄ stain. Flash column chromatography was performed using silica gel (40-63 µm particle size) purchased from Geduran^{®}.

### Photochemistry

All N-alkylation reactions with blue light were carried out using a photoreactor equipped with a blue LED module (KT-Elektronik, "100W Power LED blau 450 nm Aquarium", 450 nm, 100 W), consisting out of 100 LED-chips. The power of the LED was adjusted using a linear regulator. The vials were cooled with two Peltier-elements (TEC1-12706) while being irradiated with blue light.

### Spectroscopy and Instruments

NMR spectra were recorded on a Bruker Ascend^{™} 500 spectrometer operating at 500 MHz, 471 MHz and 126 MHz, for ¹H, ¹⁹F and ¹³C acquisitions, respectively; or on a Varian Unity/Inova 600 spectrometer operating at 600 MHz and 151 MHz for ¹H and ¹³C acquisitions, respectively. Chemical shifts are reported in ppm with the solvent residual peak as the internal standard.

### Starting materials

All substrates were used as received from commercial suppliers, unless otherwise stated. The [1.1.1]propellane solution was prepared according to the literature. Trifluoromethyl thianthrenium triflate salt (CF₃-TT⁺OTf⁻) was prepared according to the literature. Thianthrene radical cation (TT^{•+}) was prepared according to the literature. Ir(ppy)₃ was purchased from Sigma-Aldrich. lr[(dtbbpy)(ppy)₂]PF₆ was purchased from Sigma-Aldrich. CuCI was purchased from Alfa Aesar. Et₃N was purchased from Acros Organics. Ni(dtbbpy)Br₂ was prepared according to the literature. 4CzIPN was prepared according to the literature. The phenols and aryl bromides were prepared according to the literature.

### Preparation of [1.1.1]propellane stock solution

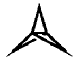

This compound was prepared following the procedure reported by Anderson:
To an oven-dried 500 mL round-bottom flask containing a teflon-coated magnetic stirring bar was added 1,1-dibromo-2,2-bis(chloromethyl)cyclopropane (16.0 g, 52.8 mmol, 1.00 equiv.). The flask was sealed with a septum-cap, evacuated, and back-filled with argon three times, and then anhydrous Et₂O (33 mL) was added. The mixture was cooled to - 45 °C (dry ice/isopropanol bath). Phenyllithium (56 mL, 1.9 M in Bu₂O, 0.11 mol, 2.0 equiv.) was added dropwise via syringe over 15 min at -45°C. The cooling bath was replaced with an ice bath, and the mixture was warmed to 0°C, and then stirred at this temperature for 2 h. Upon completion of the reaction, the mixture was then distilled at 25 °C (70 mbar) using a rotary evaporator with dry ice trap, the receiving flask of which was immersed in a dry ice/acetone bath. The [1.1.1]propellane stock solution (31 mL, 0.85 M in Et₂O, 50%) was transferred to a flame-dried septum-sealed bottle under an inert atmosphere, and stored at -20°C. The approximate concentration of the solution was determined by quantitative ¹H NMR spectroscopy with 1,2-dichloroethane as an internal standard.

### Preparation of bicyclo[1.1.1]pentane thianthrenium salts

### Trifluoromethylbicyclo[1.1.1]pentane thianthrenium salt (1)

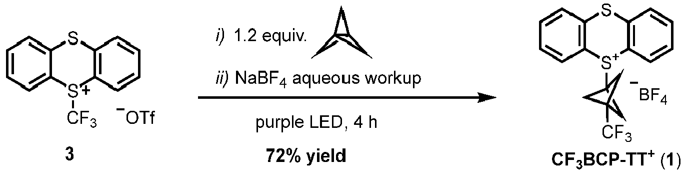

To a 500 mL round-bottom flask equipped with a stirring bar were added trifluoromethyl thianthrenium salt 3 (9.23 g, 21.2 mmol, 1.00 equiv.) and anhydrous MeCN (152 mL, 0.140 M). The flask was capped with a rubber septum, and subsequently [1.1.1]propellane solution in Et₂O (c = 0.85 M, 30 mL, 1.7 g, 1.2 equiv.) was added dropwise via syringe to the reaction over 10 min while stirring. Subsequently, the reaction flask was placed in front of two Kessil PR160-390 nm LEDs. The mixture was irradiated for 4 h while maintaining the temperature at approximately 30 °C through cooling with a fan. After 4 h, the reaction flask was removed from the two Kessil PR160-390 nm LEDs. The mixture was concentrated under reduced pressure, diluted with DCM (150 mL). The DCM solution was poured into a separation funnel and washed with aqueous NaBF₄ solution (3 × ca. 150 mL, 10% w/w). All organic phases were combined, dried over MgSO₄ (10 g), filtered, and the solvent evaporated under reduced pressure. The crude material was purified by chromatography on silica gel eluting first with 100% EtOAc and later DCM/i-PrOH (1/0-95/5 (v/v)) to afford the title compound as a brown solid. The solid material was dissolved in DCM (ca. 30 mL), and the flask containing the solid in DCM was placed in an ice bath, at which point the residue was triturated with Et₂O (ca. 200 mL). The flask was kept at 0 °C for 1h. The resulting solid was decanted and washed with ice-cold Et₂O (2 × ca. 50 mL), and dried under vacuum overnight yielding a beige solid 1 (6.73 g, 15.3 mmol, 72%).

R_{f}= 0.53 (DCM/MeOH, 10:1 (v/v)).

Melting point: 150-151 °C (recryst. solvents: DCM/Et₂O (2:1)). The melting process is accompanied by decomposition.

HRMS-ESI (m/z) calculated for C₁₈H₁₄F₃S₂⁺ [M-BF_{4]}⁺, 351.0483; found, 351.0484; deviation: +0.2 ppm.

Elemental analysis calcd (%) for C₁₈H₁₄BF₇S₂: C 49.33, H 3.22; found: C 49.09, H 3.21.

### Cyanobicyclo[1.1.1]pentane thianthrenium salt (2)

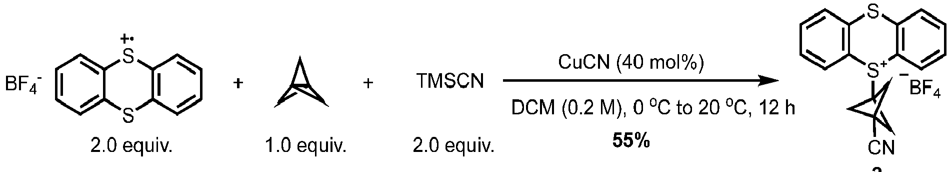

Under nitrogen atmosphere, to a 4 mL borosilicate vial equipped with a magnetic stir bar were added thianthrenium tetrafluoroborate (TT^{•+} BF₄⁻) (120 mg, 0.200 mmol, 2.00 equiv.), CuCN (7.2 mg, 80 µmoL, 40 mol%), and anhydrous DCM (1.0 mL, c = 0.2 M). The vial was sealed with a septum-cap. After cooling to 0 °C, TMSCN (50 µL, 40 mg, 0.40 mmol, 2.0 equiv.) was added in one portion. The mixture was stirred for 10 min at 0 °C. Subsequently, [1.1.1]propellane solution in Et₂O (c = 0.90 M) (0.22 mL, 0.20 mmol, 1.0 equiv.) was added to the mixture. Then, the mixture was stirred at 0 °C for 3 h, followed by stirring at 20 °C for 9 h. The mixture was diluted with MeCN (3 mL), and washed with aqueous NaBF₄ solution (1 × 3 mL, 10% w/w). The resulting mixture was poured into a separation funnel, vigorously shaken, and the layers were separated. The aqueous layer was extracted with DCM (2 × 5 mL). The combined organic phase was dried over Na₂SO₄, filtered, and the solvent was removed under reduced pressure. The residue was purified by chromatography on silica gel eluting with DCM/MeOH (1/0-30/1 (v/v)) to afford the title compound as a pale brown solid (43.4 mg, 0.110 mmol, 55%).

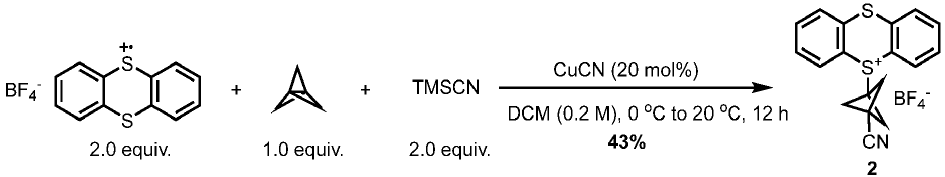

Under nitrogen atmosphere, to a 50 mL round-bottom flask equipped with a magnetic stir bar were added thianthrenium tetrafluoroborate (TT^{•+} BF₄⁻) (3.18 g, 10.5 mmol, 1.98 equiv.), CuCN (95 mg, 1.1 mmoL, 20 mol%), and anhydrous DCM (26 mL, c = 0.20 M). The flask was sealed with a septum-cap. After cooling to 0 °C, TMSCN (1.33 mL, 1.05 g, 10.6 mmol, 2.00 equiv.) was added in one portion. The mixture was stirred for 10 min at 0 °C. Subsequently, [1.1.1]propellane solution in Et₂O (c= 0.71 M) (7.5 mL, 5.3 mmol, 1.0 equiv.) was added to the mixture. Then, the mixture was stirred at 0 °C for 3 h, followed by stirring at 20 °C for 9 h. The mixture was diluted with DCM (20 mL) and MeCN (20 mL), and washed with aqueous NaBF₄ solution (1 × 20 mL, 10% w/w). The resulting mixture was poured into a separation funnel, vigorously shaken, and the layers were separated. The aqueous layer was extracted with DCM (2 × 50 mL). The combined organic phase was dried over Na₂SO₄, filtered, and the solvent was removed under reduced pressure. The residue was purified by fast chromatography on silica gel eluting with DCM/MeOH (1/0-30/1 (v/v)) to afford the title compound as a pale brown solid (890 mg, 2.25 mmol, 43%).

R_{f}= 0.47 (DCM/MeOH, 10:1 (v/v)).

Melting point: 147-148 °C

HRMS-ESI (m/z) calculated for C₁₈H₁₄NS₂⁺ [M-BF₄]⁺, 308.0560; found, 308.0562; deviation: +0.8 ppm.

### General procedure for Cu-catalyzed C-O coupling of BCP-TT⁺ BF₄⁻ with phenols

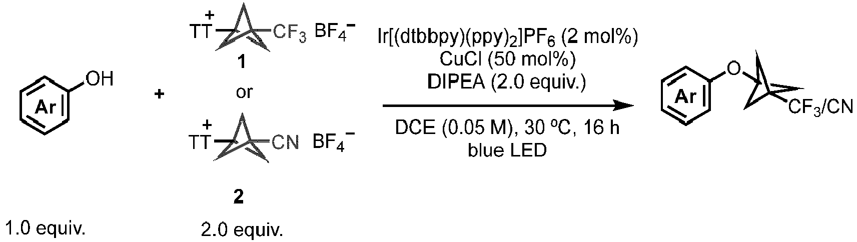

Under nitrogen atmosphere, to a 4 mL borosilicate vial equipped with a magnetic stir bar were added phenol (0.150 mmol, 1.00 equiv.), 1 or 2 (0.300 mmol, 2.00 equiv.), Ir[(dtbbpy)(ppy)₂]PF₆ (3 mg, 3 µmoL, 2 mol%), CuCl (7.4 mg, 75 µmoL, 50 mol%), anhydrous DCE (3.0 mL, c = 50 mM), and DIPEA (52 µL, 39 mg, 0.30 mmol, 2.0 equiv.). The vial was sealed with a septum-cap. Then, the mixture was stirred for 10 min at 25 °C, and placed 5 cm away from two blue LEDs (Kessil A160WE Tuna Blue, LED lighting, 40 W). The mixture was irradiated for 16 h while maintaining the temperature at approximately 30 °C through cooling with a fan. After irradiation, the mixture was concentrated under reduced pressure. The residue was purified by flash column chromatography on silica gel to afford the desired product.

Note: The reaction is air and water sensitive. Schlenk technique was used to avoid air and water. For simplicity, in the inventors'research, the inventors have opted to execute the transformation for most compounds by using a glovebox. Control experiments showed that the yields were within error of measurement if the reaction was carried out using a glovebox or Schlenk technique.

### General procedure A for Cu-catalyzed C-N coupling of 1 (BCP-TT⁺ BF₄⁻) with N-nucleophiles

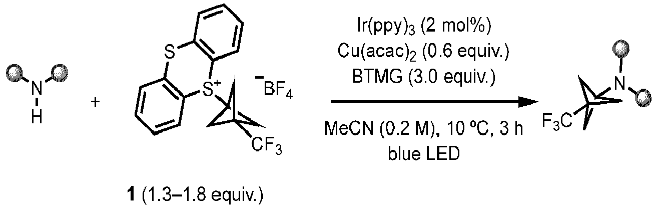

Under air, a 4 mL borosilicate vial equipped with a magnetic stir bar was charged with the N-nucleophile (0.300 mmol, 1.00 equiv.), 1 (1.30-1.70 equiv.), Ir(ppy)₃ (4 mg, 6 µmol, 2 mol%), and Cu(acac)₂ (47 mg, 0.18 mmol, 60 mol%). The vial was sealed with a septum-cap, evacuated, and flushed with argon three times using Schlenk technique. Under positive pressure of argon of 0.5 bars, 2-tert-butyl-1,1,3,3-tetramethylguanidine (BTMG) (0.18 mL, 0.15 g, 0.90 mmol, 3.0 equiv.) was added, followed by anhydrous MeCN (1.5 mL, c= 0.2 M). The mixture was irradiated for 3 h at 10 °C using a photoreactor equipped with a blue LED module (KT-Elektronik, "100W Power LED blau 450 nm Aquarium", 450 nm, 60 W), cooled with two Peltier-elements (TEC1-12706). After irradiation, the mixture was concentrated under reduced pressure. The residue was purified by flash column chromatography on silica gel to afford the desired product.

Note: The N-nucleophile was used as limiting reagent.

### General procedure B for Cu-catalyzed C-N coupling of 1 (BCP-TT⁺ BF₄⁻) with N-nucleophiles

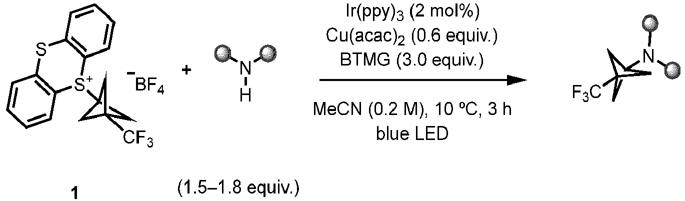

Under air, a 4 mL borosilicate vial equipped with a magnetic stir bar was charged with 1 (0.250 mmol, 1.00 equiv.), the N-nucleophile (1.50-1.80 equiv.), Ir(ppy)₃ (3.3 mg, 5.0 µmol, 2 mol%), and Cu(acac)₂ (39.3 mg, 0.150 mmol, 60 mol%). The vial was sealed with a septum-cap, evacuated, and flushed with argon three times using Schlenk technique. Under positive pressure of argon of 0.5 bars, 2-tert-butyl-1,1,3,3-tetramethylguanidine (BTMG) (0.15 mL, 0.13 g, 0.75 mmol, 3.0 equiv.) was added, followed by anhydrous MeCN (1.25 mL, c = 0.200 M). The mixture was irradiated for 3 h at 10 °C using a photoreactor equipped with a blue LED module (KT-Elektronik, "100W Power LED blau 450 nm Aquarium", 450 nm, 60 W), cooled with two Peltier-elements (TEC1-12706). After irradiation, the mixture was concentrated under reduced pressure. The residue was purified by flash column chromatography on silica gel to afford the desired product.

Note: The reagent 1 was used as limiting reagent.

### General procedure for Ni-catalyzed reductive C-C coupling of 1 (BCP-TT⁺ BF₄⁻) with aryl bromides

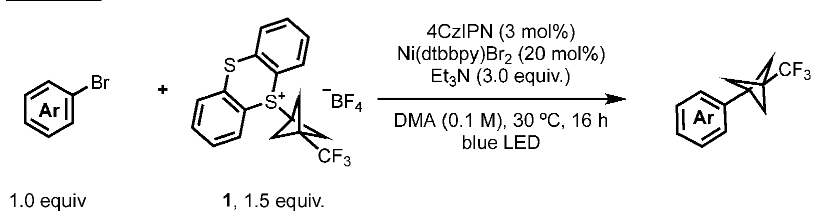

Under nitrogen atmosphere, to a 4 mL borosilicate vial equipped with a magnetic stir bar were added aryl bromide (0.200 mmol, 1.00 equiv.), 1 (130 mg, 0.300 mmol, 1.50 equiv.), 4CzIPN (5 mg, 6 µmoL, 3 mol%), Ni(dtbbpy)Br₂ (19.4 mg, 40.0 µmoL, 20.0 mol%), anhydrous DMA (2.0 mL, c= 0.10 M), and Et₃N (83 µL, 61 mg, 0.60 mmol, 3.0 equiv.). The vial was sealed with a septum-cap. Then, the mixture was stirred for 10 min at 25 °C, and placed 5 cm away from two blue LEDs (Kessil A160WE Tuna Blue, LED lighting, 40 W). The mixture was irradiated for 16 h while maintaining the temperature at approximately 30 °C through cooling with a fan. After irradiation, EtOAc (6 mL) was added to the mixture, and washed with brine (2 × 3 mL). The organic phase was dried over Na₂SO₄, filtered, and the solvent was removed under reduced pressure. The residue was purified by flash column chromatography on silica gel to afford the desired product.

### Bicyclo[1.1.1]pentylether 8

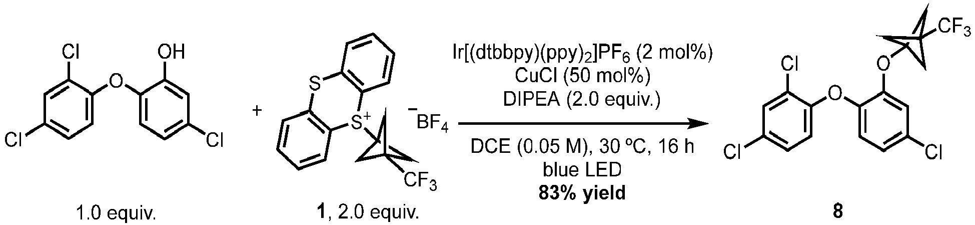

Under nitrogen atmosphere, to a 4 mL borosilicate vial equipped with a magnetic stir bar were triclosan (45.2 mg, 0.150 mmol, 1.00 equiv.), 1 (130 mg, 0.300 mmol, 2.00 equiv.), lr[(dtbbpy)(ppy)₂]PF₆ (3 mg, 3 µmoL, 2 mol%), CuCl (7.4 mg, 75 µmoL, 50 mol%),anhydrous DCE (3.0 mL, c = 50 mM), and DIPEA (52 µL, 39 mg, 0.30 mmol, 2.0 equiv.). The vial was sealed with a septum-cap. Then, the mixture was stirred for 10 min at 25 °C, and placed 5 cm away from two blue LEDs (Kessil A160WE Tuna Blue, LED lighting, 40 W). The mixture was irradiated for 16 h while maintaining the temperature at approximately 30 °C through cooling with a fan. After irradiation, the mixture was concentrated under reduced pressure. The residue was purified by flash column chromatography on silica gel eluting with pentane to afford the title compound 8 as a colorless oil (52.9 mg, 0.125 mmol, 83%).

R_{f}= 0.50 (pentanes/EtOAc, 95:5 (v/v)).

HRMS-EI (m/z) calc'd for C₁₈H₁₂O₂F₃Cl₃⁺ [M]⁺, 421.9855; found, 421.9849; deviation: -1.2 ppm.

### Bicyclo[1.1.1]pentylether 9

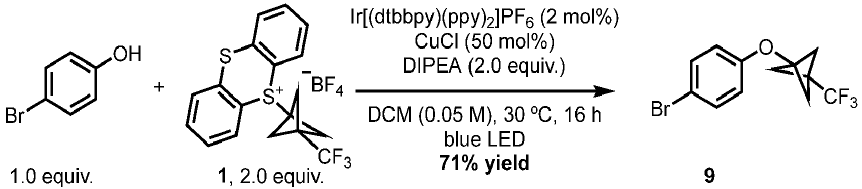

Under nitrogen atmosphere, to a 4 mL borosilicate vial equipped with a magnetic stir bar were added 4-bromo phenol (31.1 mg, 0.180 mmol, 1.00 equiv.), 1 (160 mg, 0.360 mmol, 2.00 equiv.), Ir[(dtbbpy)(ppy)₂]PF₆ (3 mg, 3 µmoL, 2 mol%), CuCI (8.9 mg, 90 µmoL, 50 mol%), anhydrous DCE (3.0 mL, c = 60 mM), and DIPEA (63 µL, 47 mg, 0.36 mmol, 2.0 equiv.). The vial was sealed with a septum-cap. Then, the mixture was stirred for 10 min at 25 °C, and placed 5 cm away from two blue LEDs (Kessil A160WE Tuna Blue, LED lighting, 40 W). The mixture was irradiated for 16 h while maintaining the temperature at approximately 30 °C through cooling with a fan. After irradiation, the mixture was concentrated under reduced pressure. The residue was purified by flash column chromatography on silica gel eluting with EtOAc/pentane (0:100-1:50 (v/v)) to afford the title compound 9 as a yellow oil (39.2 mg, 0.128 mmol, 71%).

R_{f}= 0.50 (pentane/EtOAc, 20:1 (v/v)).

HRMS-Cl (m/z) calc'd for C₁₂H₁₁OF₃Br⁺ [M+H]⁺, 306.9939; found, 306.9940; deviation: +0.4 ppm.

### Bicyclo[1.1.1]pentylether 13

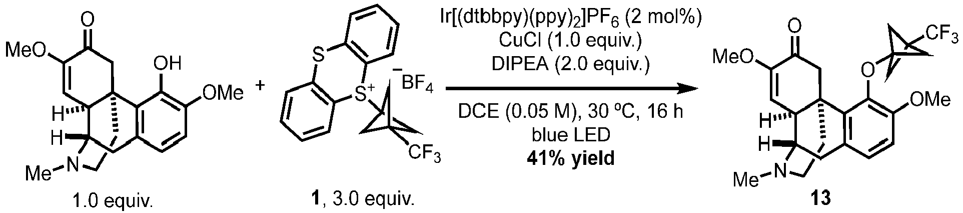

Under nitrogen atmosphere, to a 4 mL borosilicate vial equipped with a magnetic stir bar were added sinomenine (32.9 mg, 0.100 mmol, 1.00 equiv.), 1 (130 mg, 0.300 mmol, 3.00 equiv.), lr[(dtbbpy)(ppy)₂]PF₆ (2 mg, 2 µmoL, 2 mol%), CuCI (9.9 mg, 0.10 mmoL, 1.0 equiv), anhydrous DCE (2.0 mL, c = 50 mM), and DIPEA (35 µL, 26 mg, 0.20 mmol, 2.0 equiv.). The vial was sealed with a septum-cap. Then, the mixture was stirred for 10 min at 25 °C, and placed 5 cm away from two blue LEDs (Kessil A160WE Tuna Blue, LED lighting, 40 W). The mixture was irradiated for 16 h while maintaining the temperature at approximately 30 °C through cooling with a fan. After irradiation, the mixture was concentrated under reduced pressure. The residue was purified by flash column chromatography on silica gel eluting with MeOH/DCM (0:100-1:20 (v/v)) to afford the title compound 13 as a yellow oil (19.0 mg, 41.0 µmol, 41%).

R_{f} = 0.30 (MeOH/DCM, 1:10 (v/v)).

HRMS-ESI (m/z) calc'd for C₂₆H₂₉NO₄F₃⁺ [M+H]⁺, 464.2043; found, 464.2046; deviation: +0.6 ppm.

### Bicyclo[1.1.1]pentylether 26

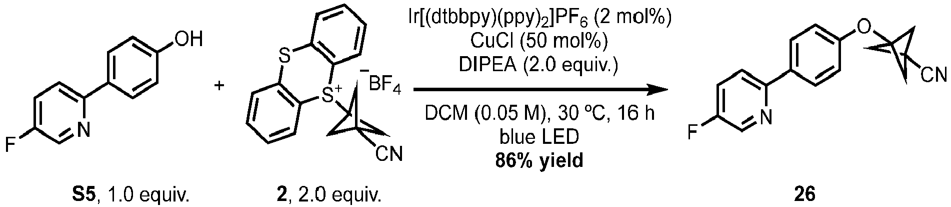

Under nitrogen atmosphere, to a 4 mL borosilicate vial equipped with a magnetic stir bar were added 4-(5-fluoropyridin-2-yl)phenol (18.9 mg, 0.100 mmol, 1.00 equiv.), 2 (79.0 mg, 0.200 mmol, 2.00 equiv.), lr[(dtbbpy)(ppy)₂]PF₆ (2 mg, 2 µmoL, 2 mol%), CuCI (4.9 mg, 50 µmoL, 50 mol%), anhydrous DCM (2.0 mL, c = 0.05 M), and DIPEA (35 µL, 26 mg, 0.20 mmol, 2.0 equiv.). The vial was sealed with a septum-cap. Then, the mixture was stirred for 10 min at 25 °C, and placed 5 cm away from two blue LEDs (Kessil A160WE Tuna Blue, LED lighting, 40 W). The mixture was irradiated for 16 h while maintaining the temperature at approximately 30 °C through cooling with a fan. After irradiation, the mixture was concentrated under reduced pressure. The residue was purified by flash column chromatography on silica gel eluting with EtOAc/pentane (0:100-1:8 (v/v)) to afford the title compound 26 as a colorless oil (24.0 mg, 86.0 µmol, 86%).

R_{f}= 0.50 (pentane/EtOAc, 3:1 (v/v)).

HRMS-EI (m/z) calc'd for C₁₇H₁₃N₂OF⁺ [M]⁺, 280.1007; found, 280.1006; deviation: +0.1 ppm.

### Bicyclo[1.1.1]pentylamine 43

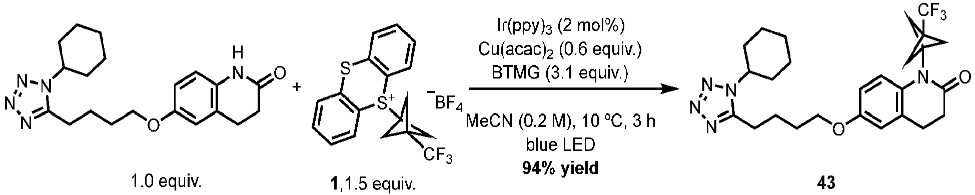

A 4 mL borosilicate vial equipped with a magnetic stir bar was charged with cilostazol ( 0.11 g, 0.30 mmol, 1.0 equiv.), 1 (0.20 g, 4.5 mmol, 1.5 equiv.), Ir(ppy)₃ (4 mg, 6 µmol, 2 mol%), and Cu(acac)₂ (47 mg, 0.18 mmol, 0.60 equiv.). The vial was sealed with a septum-cap, evacuated, and flushed with argon three times using Schlenk technique. Under positive pressure of argon, anhydrous MeCN (1.5 mL, c = 0.2 M) was added, followed by *2-tert-*butyl-1,1,3,3-tetramethylguanidine (0.19 mL, 0.16 g, 0.94 mmol, 3.1 equiv.) while stirring. The mixture was irradiated for 3 h at 10 °C using a photoreactor equipped with a blue LED module (KT-Elektronik, "100W Power LED blau 450 nm Aquarium", 450 nm, 60 W), cooled with two Peltier-elements (TEC1-12706). Then, the mixture was concentrated to dryness.

The residue was purified by chromatography on silica gel eluting with EtOAc/hexanes (3:10-9:20 (v/v)) to afford the title compound 43 as a brown solid (142 mg, 0.281 mmol, 94%).

R_{f}= 0.30 (EtOAc/hexanes, 3:2 (v/v)).

HRMS-ESI (m/z) calculated for C₂₆H₃₂N₅O₂F₃Na⁺ [M+Na]⁺, 526.2407; found, 526.2400; deviation: -1.2 ppm.

### Bicyclo[1.1.1]pentylamine 46

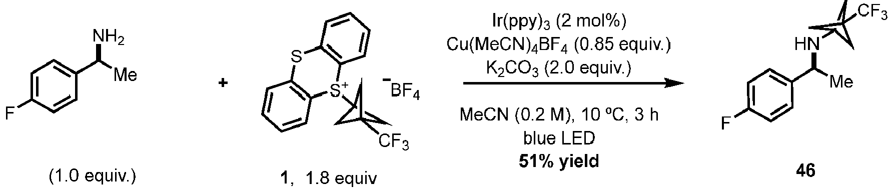

A 4 mL borosilicate vial equipped with a magnetic stir bar was charged with (S)-4-fluoro-α-methylbenzylamine (40.0 µL, 0.300 mmol, 1.00 equiv.), 1 (236 mg, 0.538 mmol, 1.80 equiv.), Ir(ppy)₃ (6.0 mg, 9.2 µmol, 2.0 mol%), K₂CO₃ (83 mg, 0.60 mol, 2.0 equiv., and Cu(MeCN)₄BF₄ (80.5 mg, 0.256 mmol, 0.853 equiv.). The vial was sealed with a septum-cap, evacuated, and flushed with argon three times using Schlenk technique. Under positive pressure of argon, anhydrous MeCN (1.5 mL, c = 0.2 M) was added while stirring. The mixture was irradiated for 3 h at 10 °C using a photoreactor equipped with a blue LED module (KT-Elektronik, "100W Power LED blau 450 nm Aquarium", 450 nm, 60 W), cooled with two Peltier-elements (TEC1-12706). Then, the mixture was concentrated to dryness. The residue was purified by chromatography on silica gel eluting with EtOAc/hexanes (5:100-3:25 (v/v)) to afford the title compound 46 as a yellow liquid (42.0 mg, 0.154 mmol, 51%).

R_{f} = 0.30 (EtOAc/hexanes, 1:5 (v/v)).

HRMS-EI (m/z) calculated for C₁₄H₁₅NF₄⁺ [M]⁺, 273.1135; found, 273.1135; deviation: +0.2 ppm.

### Bicyclo[1.1.1]pentylamine 47

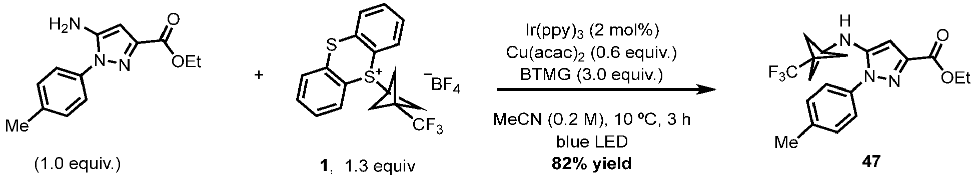

A 4 mL borosilicate vial equipped with a magnetic stir bar was charged with ethyl-5-amino-1-(4-methylphenyl)-1H-pyrazol-4-carboxylate (40.0 mg, 0.163 mmol, 1.00 equiv.), 1 (92.9 mg, 0.212 mmol, 1.30 equiv.), Ir(ppy)₃ (2.1 mg, 3.3 µmol, 2.0 mol%), and Cu(acac)₂ (25.6 mg, 0.098 mmol, 0.600 equiv.). The vial was sealed with a septum-cap, evacuated, and flushed with argon three times using Schlenk technique. Under positive pressure of argon, anhydrous MeCN (1.0 mL, c = 0.2 M) was added, followed by 2-tert-butyl-1,1,3,3-tetramethylguanidine (0.10 mL, 84 mg, 0.49 mmol, 3.0 equiv.) while stirring. The mixture was irradiated for 3 h at 10 °C using a photoreactor equipped with a blue LED module (KT-Elektronik, "100W Power LED blau 450 nm Aquarium", 450 nm, 60 W), cooled with two Peltier-elements (TEC1-12706). Then, the mixture was concentrated to dryness. The residue was purified by chromatography on silica gel eluting with hexanes/EtOAc (1/0-1/4 (v/v)) to afford the title compound 47 as a light yellow oil (51.0 mg, 0.134 mmol, 82%).

R_{f}= 0.80 (EtOAc/hexanes, 1:1 (v/v)).

HRMS-ESI (m/z) calculated for C₁₉H₂₁N₃F₃O₂⁺ [M+H]⁺, 380.1579; found, 380.1580; deviation: +0.2 ppm.

### Bicyclo[1.1.1]pentylamine 53

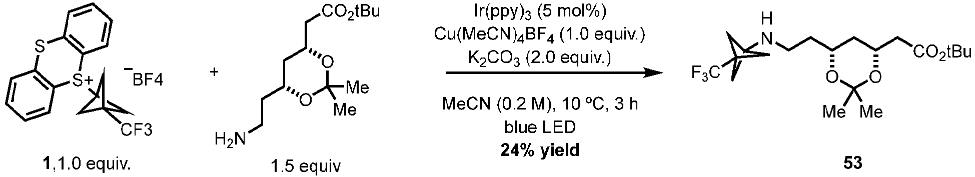

A 4 mL borosilicate vial equipped with a magnetic stir bar was charged with 1 (131 mg, 0.300 mmol, 1.00 equiv.), tert-butyl 2-(4R-cis)-6-aminoethyl-2,2-dimethyl-1,3-dioxane-4-acetate (123 mg, 0.450 mmol, 1.50 equiv.), Ir(ppy)₃ (10 mg, 15 µmol, 5.0 mol%), K₂CO₃ (83 mg, 0.60 mol, 2.0 equiv), and Cu(MeCN)₄BF₄ (94 mg, 0.30 mmol, 1.0 equiv.). The vial was sealed with a septum-cap, evacuated, and flushed with argon three times using Schlenk technique. Under positive pressure of argon, anhydrous MeCN (1.5 mL, c = 0.2 M) was added while stirring. The mixture was irradiated for 3 h at 10 °C using a photoreactor equipped with a blue LED module (KT-Elektronik, "100W Power LED blau 450 nm Aquarium", 450 nm, 60 W), cooled with two Peltier-elements (TEC1-12706). Then, the mixture was concentrated to dryness. The residue was purified by chromatography on silica gel eluting with EtOAc/hexanes (10:100-3:25 (v/v)) to afford the title compound 53 as a yellow liquid (29 mg, 0.07 mmol, 24%).

R_{f}= 0.40 (EtOAc/hexanes, 1:1 (v/v)).

HRMS-ESI (m/z) calculated for C₂₀H₃₃NF₃O₄⁺ [M+H]⁺, 408.2358; found, 408.2356; deviation: -0.3 ppm.

### Bicyclo[1.1.1]pentylarene 68

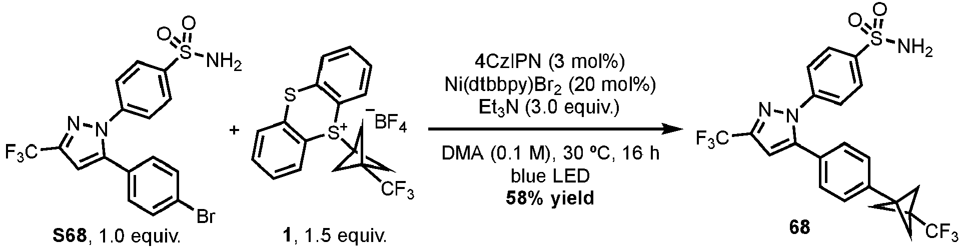

Under nitrogen atmosphere, to a 4 mL borosilicate vial equipped with a magnetic stir bar were added S68 (89.2 mg, 0.200 mmol, 1.00 equiv.), 1 (130 mg, 0.300 mmol, 1.50 equiv.), 4CzIPN (5 mg, 6 µmoL, 3 mol%), Ni(dtbbpy)Br₂ (19.4 mg, 40.0 µmoL, 20.0 mol%), anhydrous DMA (2.0 mL, c = 0.10 M), and Et₃N (83 µL, 61 mg, 0.60 mmol, 3.0 equiv.). The vial was sealed with a septum-cap. Then, the mixture was stirred for 10 min at 25 °C, and placed 5 cm away from two blue LEDs (Kessil A160WE Tuna Blue, LED lighting, 40 W). The mixture was irradiated for 16 h while maintaining the temperature at approximately 30 °C through cooling with a fan. After irradiation, EtOAc (6 mL) was added to the mixture, and washed with brine (2 × 3 mL). The organic phase was dried over Na₂SO₄, filtered, and the solvent was removed under reduced pressure. The residue was purified by flash column chromatography on silica gel eluting with EtOAc/pentane (0:100-1:6 (v/v)) to afford the title compound 68 as a colorless solid (58.2 mg, 0.116 mmol, 58%).

R_{f}= 0.54 (pentane/EtOAc, 3:1 (v/v)).

HRMS-ESI (m/z) calc'd for C₂₂H₁₆N₃O₂S₁F₆ [M-H]⁻, 500.0877; found, 500.0873; deviation: - 0.7 ppm.

### Bicyclo[1.1.1]pentylarene 73

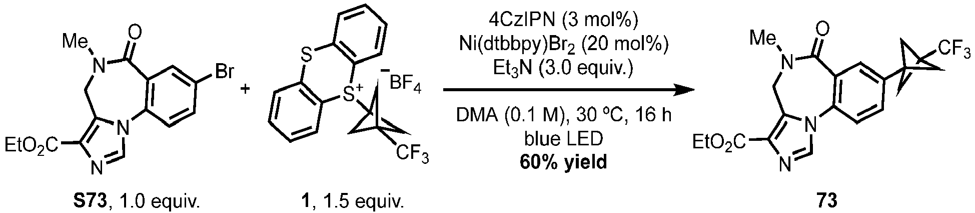

Under nitrogen atmosphere, to a 4 mL borosilicate vial equipped with a magnetic stir bar were added S73 (72.6 mg, 0.200 mmol, 1.00 equiv.), 1 (130 mg, 0.300 mmol, 1.50 equiv.), 4CzIPN (5 mg, 6 µmoL, 3 mol%), Ni(dtbbpy)Br₂ (19.4 mg, 40.0 µmoL, 20.0 mol%), anhydrous DMA (2.0 mL, c= 0.10 M), and Et₃N (83 µL, 61 mg, 0.60 mmol, 3.0 equiv.). The vial was sealed with a septum-cap. Then, the mixture was stirred for 10 min at 25 °C, and placed 5 cm away from two blue LEDs (Kessil A160WE Tuna Blue, LED lighting, 40 W). The mixture was irradiated for 16 h while maintaining the temperature at approximately 30 °C through cooling with a fan. After irradiation, EtOAc (6 mL) was added to the mixture, and washed with brine (2 × 3 mL). The organic phase was dried over Na₂SO₄, filtered, and the solvent was removed under reduced pressure. The residue was purified by flash column chromatography on silica gel eluting with DCM/MeOH (100:0-100:1 (v/v)) to afford the title compound 73 as a colorless solid (49.9 mg, 0.119 mmol, 60%).

R_{f}= 0.30 (DCM/MeOH, 25:1 (v/v)).

HRMS-ESI (m/z) calc'd for C₂₁H₂₀F₃N₃O₃Na⁺ [M+Na]⁺, 442.1353; found, 442.1349; deviation: -0.9 ppm.

### Bicyclo[1.1.1]pentylmetaxalone 75

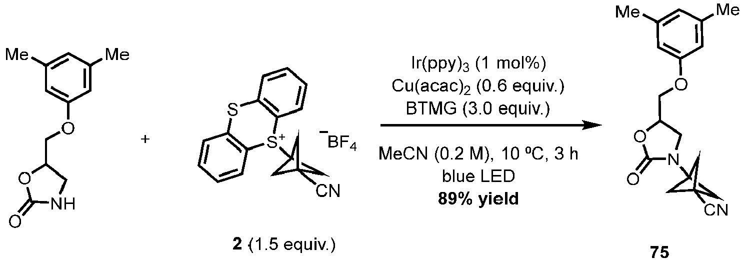

A 4 mL borosilicate vial equipped with a magnetic stir bar was charged with metaxalone (22.1 mg, 0.100 mmol, 1.00 equiv.), **2** (59.3 mg, 0.150 mmol, 1.50 equiv.), Ir(ppy)₃ (0.7 mg, 1 µmol, 1 mol%), and Cu(acac)₂ (15.5 mg, 60.0 µmol, 0.60 equiv.). The vial was sealed with a septum-cap, evacuated, and flushed with argon three times using Schlenk technique. Under positive pressure of argon, 2-tert-butyl-1,1,3,3-tetramethylguanidine (61 µL, 51 mg, 0.30 mmol, 3.0 equiv.) was added, followed by anhydrous MeCN (0.5 mL, c = 0.2 M). The mixture was irradiated for 3 h at 10 °C using a photoreactor equipped with a blue LED module (KT-Elektronik, "100W Power LED blau 450 nm Aquarium", 450 nm, 60 W), cooled with two Peltier-elements (TEC1-12706). Then, the mixture was concentrated to dryness. The residue was purified by chromatography on silica gel eluting with EtOAc/pentane (1/5-2/1 (v/v)) to afford the title compound **xx** as a pale yellow oil (27.8 mg, 89.0 µmol, 89%).

R_{f}= 0.57 (pentane/EtOAc, 1:1 (v/v)).

HRMS-EI (m/z) calculated for C₁₈H₂₀N₂O₃⁺ [M]⁺, 312.1471; found, 312.1468; deviation: - 0.7 ppm.

### Bicyclo[1.1.1]pentylarene 77

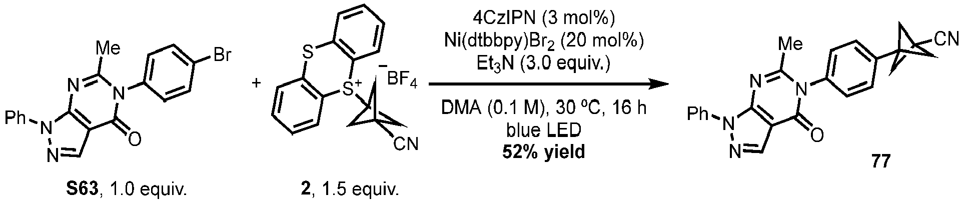

Under nitrogen atmosphere, to a 4 mL borosilicate vial equipped with a magnetic stir bar were added **S63** (38.1 mg, 0.100 mmol, 1.00 equiv.), **2** (59.3 mg, 0.150 mmol, 1.50 equiv.), 4CzIPN (2.5 mg, 3.0 µmoL, 3 mol%), Ni(dtbbpy)Br₂(9.7 mg, 20 µmoL, 20 mol%), anhydrous DMA (1.0 mL, c = 0.10 M), and Et₃N (42 µL, 30 mg, 0.30 mmol, 3.0 equiv.). The vial was sealed with a septum-cap. Then, the mixture was stirred for 10 min at 25 °C, and placed 5 cm away from two blue LEDs (Kessil A160WE Tuna Blue, LED lighting, 40 W). The mixture was irradiated for 16 h while maintaining the temperature at approximately 30 °C through cooling with a fan. After irradiation, EtOAc (6 mL) was added to the mixture, and washed with brine (2 × 3 mL). The organic phase was dried over Na₂SO₄, filtered, and the solvent was removed under reduced pressure. The residue was purified by flash column chromatography on silica gel eluting with EtOAc/pentane (0:100-1:5 (v/v)) to afford the title compound **xx** as a colorless solid (20.4 mg, 51.8 µmol, 52%).

**R_{f}=** 0.45 (pentane/EtOAc, 2:1 (v/v)).

**HRMS-ESI (m/z)** calc'd for C₂₄H₁₉N₅ONa⁺ [M+Na]⁺, 416.1480; found, 416.1482; deviation: +0.4 ppm.

As discussed above, the incorporation of three-dimensional small-ring scaffolds into bioactive molecules can improve their pharmacokinetic profile, including increased metabolic stability and solubility. Therefore, the rigid 1,3-disubstituted bicyclo[1.1.1]pentanes (BCPs) have shown promise as linear bioisosteres for *para-*substituted benzene rings in drug development. Construction of BCPs commonly requires the cumbersome use of a volatile and labile [1.1.1]propellane solution as reagent, and more stable reagents do not show the versatile reactivity of propellane itself. The lack of practical reagents for BCP introduction currently impedes the potential of this promising scaffold for *pharmaceutical* development. Here, the present inventors report stable thianthrenium-based BCP reagents for practical oxygen-, nitrogen-, and carbon alkylation reactions that expand the bicyclopentylation scope of any other reagent, including [1.1.1]propellane; aryl BCP ethers are characterized as a new structural motif in chemistry.

The redox and stereoelectronic properties of the thianthrene scaffold are relevant for both the synthesis via strain release as well as the subsequent reactivity of the new reagents. The weak carbon-sulfur bond can undergo selective mesolytic cleavage upon single-electron reduction to produce BCP radicals that engage in transition-metal-mediated bond formations, including alkylation of phenols, N-heterocycles, amines, amides, sulfonamides, anilines, arenes, and heteroarenes, even at a late-stage with a wide variety of functional groups present. Because the versatile BCP thianthrenium reagents can be prepared centrally and are stable to storage and transport in ambient conditions, they display potential for practical applications in pharmaceutical research for exploration of high-value structures that otherwise may not have been accessed.

## Claims

1. A thianthrene derivative of the Formula (I): 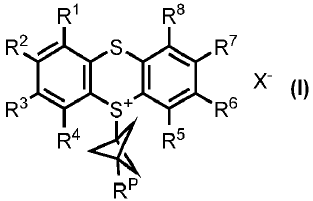 wherein R¹ to R⁸ may be the same or different and are each selected from hydrogen, halogen, a C₁ to C₆ alkyl group, which is optionally substituted by at least one halogen, or a -O-C₁ to C₆ alkyl group, wherein R^{P} represents CF₃ or CN and wherein X⁻ is an anion, selected from F-, Cl⁻, triflate-, BF₄⁻, SbF₆⁻, PF₆⁻, ClO₄⁻, 0.5 SO₄²⁻ or NO_{3.}

2. A thianthrene derivative of the Formula (I) as clamed in claim 1 wherein, in Formula (I), R¹ to R⁸ may be the same or different and are each selected from hydrogen, Cl or F, R^{p} is as defined in claim 1 and X⁻ is an anion as defined in claim 1.

3. A thianthrene derivative of the Formula (I) as clamed in claim 1 wherein, in Formula (I), R¹ to R⁸ are each hydrogen, R^{p} is as defined in claim 1 and X⁻ is an anion as defined in claim 1.

4. A thianthrene derivative of the Formula (I) as clamed in any of claims 1 to 3 wherein, in Formula (I), R¹ to R⁸ are each hydrogen, R^{p} is as defined in claim 1 and X⁻ is an anion selected from triflate or BF₄⁻.

5. Use of a bicyclopentyl thianthrenium compound of the Formula (I) as claimed in any one of claims 1 to 4 as a transfer agent for transferring a bicyclopentyl group to an organic compound selected from phenols, N-nucleophiles and aryl halogenides.

6. Use of a bicyclopentyl thianthrenium compound of the Formula (I) as claimed in any one of claims 1 to 4 as a transfer agent for transferring a bicyclopentyl group in the presence of a photocatalyst in a transition-metal-mediated bond formation to an organic compound selected from aryl halogenides, phenols and nucleophilic compounds including N-nucleophiles which organic compound is optionally substituted by at least group selected from hydroxyl, aldehyde, carboxylic acid ester, olefin, amino, amido, sulfonamido, halogen selected from bromo, fluoro, and chloro.
